# EUROPEAN PATENT SPECIFICATION

(11) **EP 2 171 098 B1**
(45) Date of publication and mention of the grant of the patent: **28.03.2018**
(21) Application number: 08781182.4
(22) Date of filing: 30.06.2008
(51) Int. Cl.: G01N 33/543, G01N 33/50

(54) **METHODS FOR EXTRACTION AND PURIFICATION OF COMPONENTS OF BIOLOGICAL SAMPLES**
VERFAHREN ZUR EXTRAKTION UND AUFREINIGUNG VON BESTANDTEILEN BIOLOGISCHER PROBEN
PROCÉDÉ D'EXTRACTION ET DE PURIFICATION DE COMPOSANTS D'ÉCHANTILLONS BIOLOGIQUES

(30) Priority: 29.06.2007 US 929512 P; 02.07.2007 US 929544 P
(43) Date of publication of application: 07.04.2010
(73) Proprietor: Becton, Dickinson and Company, Franklin Lakes, NJ 07417-1880 (US)
(72) Inventor: COLLIS, Matthew P., Thomasville, PA 17364-9789 (US); LIZZI, Michael, Stewartstown, Pennsylvania 17363 (US)
(74) Representative: dompatent von Kreisler Selting Werner - Partnerschaft von Patent- und Rechtsanwälten mbB
(86) International application number: PCT/US2008/068807
(87) International publication number: WO 2009/006417

(56) References cited:
- US-A1- 2007 031 880
- US-A1- 2007 031 880
- US-A1- 2007 092 403
- US-A1- 2007 092 403
- HOLSCHUH K ET AL: "Preparative purification of antibodies with protein A-an alternative to conventional chromatography", JOURNAL OF MAGNETISM AND MAGNETIC MATERIALS, ELSEVIER SCIENCE PUBLISHERS, AMSTERDAM, NL, vol. 293, no. 1, 1 May 2005 (2005-05-01), pages 345-348, XP027843045, ISSN: 0304-8853 [retrieved on 2005-05-01]

## Description

### FIELD OF THE INVENTION

The present invention relates generally to compositions and methods useful for the extraction of biological materials, in particular nucleic acids from biological samples. More specifically, the present invention relates to the separation of nucleic acids from biological samples.

### BACKGROUND OF THE INVENTION

In the following discussion certain articles and methods will be described for background and introductory purposes. Nothing contained herein is to be construed as an "admission" of prior art. Applicants expressly reserve the right to demonstrate, where appropriate, that the articles and methods referenced herein do not constitute prior art under the applicable statutory provisions.

In diagnostic and biochemical methodologies, access to extracted or purified cellular components, such as nucleic acids, and access to extracted or purified forms of proteins is imperative. Access to nucleic acids is required in such methodologies as nucleic acid sequencing, direct detection of particular nucleic acid sequences by nucleic acid hybridization and nucleic acid sequence amplification techniques. Therefore, a method for extracting and purifying nucleic acids should be simple, rapid and require little, if any, additional sample manipulation to gain the desired access to the nucleic acid. A method with all of these features would be extremely attractive in the automation of sample preparation, a goal of research and diagnostic laboratories. Access to purified forms of proteins is achieved through such techniques as exclusion chromatography, ion exchange chromatography, differential precipitation and the like. These methodologies, however, are troublesome for various reasons. For example, precipitation techniques are still crude and difficult to automate, and often result in unacceptable loss of sample, while chromatography is expensive and time consuming.

Effective methods for purification and manipulation of nucleic acids using paramagnetic particles are disclosed in U.S. Patents 5,973,138 ("'138") and 6,433,160 ("'160"). The paramagnetic particles used therein, reversibly bind to nucleic acids in the biological samples and allow for separation of the nucleic acids from some of the other components in the biological samples. Once separated, the bound nucleic acids are removed from the paramagnetic particles via an elution/neutralization buffer. The paramagnetic particles are then removed from the elution/neutralization buffer containing the nucleic acids. The buffer containing the nucleic acids may be used in further manipulation of the separated nucleic acids, such as hybridization, restriction, labeling, reverse transcription and amplification.

Protein purification by rapid fractionation from crude biological samples is disclosed in U.S. Pre-Grant Publication 2006-0030056 ("'0056"). Proteins in biological samples are separated by reversibly binding a protein molecule in a biological sample to a paramagnetic particle. The sample may be further processed to obtain a protein sample in a more pure form or a sample depleted of select proteins. A method that would increase the separation and isolation of components or biological samples, such as nucleic acids and proteins, from the sample would improve the product available for diagnostic and biochemical methodologies.
US 2007/031880 discloses a method for extracting components of a biological sample comprising a step of removing the component from the paramagnetic particle by adding an elution buffer and a neutralizing buffer. It describes the addition of elution buffer and neutralizing buffer in the same step.
US 2007/092403 discloses similar methods for isolating RNA or DNA from biological samples.

### SUMMARY OF INVENTION

The present invention is directed to a method of extraction of components of biological samples.

In particular, the present invention relates to a method for extracting nucleic acid components of a biological sample, comprising:
(i) in a container, reversibly binding at least one nucleic acid component of the biological sample to at least one paramagnetic particle, said paramagnetic particle being an iron oxide particle in an acid environment;
(ii) separating the at least one paramagnetic particle bound nucleic acid component from unbound components of the biological sample;
(iii) washing the at least one paramagnetic particle bound nucleic acid component;
(iv) separating the at least one paramagnetic particle bound nucleic acid component from the wash;
(v) removing the at least one nucleic acid component from the at least one paramagnetic particle by a two-step elution and neutralization process comprising
   - eluting the at least one paramagnetic particle bound nucleic acid component with a basic pH elution buffer solution consisting of potassium hydroxide or sodium hydroxide, thereby yielding an eluted sample; and
   - followed by neutralizing the eluted sample by subsequently adding a neutralizing buffer selected from bicine, Tris, 2-(cyclohexylamino)ethanesulfonic acid (CHES), N,N-bis(2-hydroxyethyl)-2-aminoethanesulfonic acid (BES), 4-morpholinepropanesulfonic acid (MOPS) or phosphate, thereby yielding an optimized buffer,
(vi) followed by separating and removing the paramagnetic particles after neutralization of the sample while the pH-optimized solution containing the unbound nucleic acid is transferred for further manipulation,
   wherein the removing step is separate from the neutralizing step.

Preferred embodiments of the present invention are apparent from the dependant claims.

The present invention provides a method for extracting nucleic acid components of biological samples that is simple, rapid and requires little, if any, additional sample manipulation.

The present invention provides a method that would increase the efficiency of separation and isolation of nucleic acid components of a biological sample.

The present invention provides improved processes for optimizing extraction of nucleic acid components of biological samples. These optimized extraction processes significantly increase the capability of separating and recovering nucleic acid components for further diagnostic and biochemical methodologies.

Embodiments of the present invention provide a method of extracting and nucleic acid components from biological samples using pH adjustment of buffers for elution and neutralization of target biological components.

Also disclosed are kits for carrying out the method of extraction and purification of components of a biological sample, such as biological molecules, organelles, and cells from biological samples.

### BRIEF DESCRIPTION OF THE DRAWINGS

Figure 1 is a graphic representation of the results of Example 7.
Figure 2 is a graphic representation of the results of Example 7.
Figure 3 is a graphic representation of the results of Example 7.
Figure 4 is a graphic representation of the results of Example 7.
Figure 5 is a graphic representation of the results of Example 7.
Figure 6 is a graphic representation of the results of Example 7.
Figure 7 is a graphic representation of the results of Example 7.
Figure 8 is a graphic representation of the results of Example 7.
Figure 9 is a graphic representation of the results of Example 8.
Figure 10 is a graphic representation of the results of Example 8.
Figure 12 is a graphic representation of the results of Example 8.

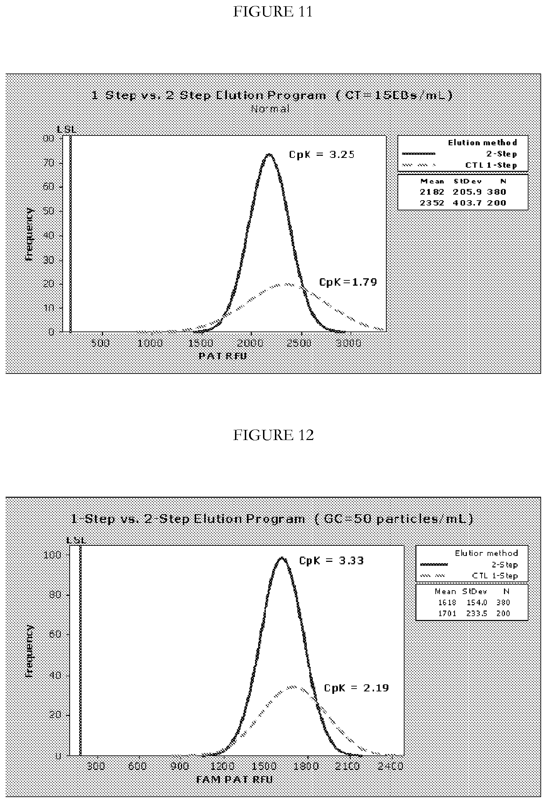

Figure 12 is a graphic representation of the results of Example 8.

### DETAILED DESCRIPTION OF THE INVENTION

The present invention is directed generally to methods for extraction of components of biological samples. In particular, the present invention describes a method of extracting a nucleic acid from a biological sample, wherein the extracted nucleic acid may be further manipulated by such means as hybridization, restriction, labeling, reverse transcription and amplification methodologies. The methods described herein present improved processes for optimizing extraction of nucleic acids from biological samples. These optimized extraction processes significantly increase the separation and recovery of nucleic acids for further diagnostic and biochemical methodologies.

As used herein, the terms "purifying" and "purification" also include extracting/extraction, isolating/isolation and concentrating/concentration and do not require absolute purity, but instead only require removal of some of or all of at least one of the components of the biological sample. In practice it is presumed that practitioners will purify to 80% or more, preferably 80%, 90%, 95% or greater purity.

The biological samples used according to the present invention, for example, clinical, forensic or environmental samples, may be any biological material, preferably containing nucleic acid. These samples may contain any viral or cellular material, including prokaryotic and eukaryotic cells, viruses, bacteriophages, mycoplasms, protoplasts and organelles, or any parts thereof. A component of a biological sample as used herein may be any part of the sample, including biological material and biological molecule(s). Such biological materials may comprise all types of mammalian and non-mammalian animal cells, plant cells, algae (including blue-green algae), fungi, bacteria, yeast, protozoa and viruses. Embodiments of this invention are used to extract nucleic acids of these compositions. Representative examples of biological materials include blood and blood-derived products such as whole blood, plasma and serum; clinical specimens such as semen, urine, feces, sputa, tissues, cell cultures and cell suspensions, nasopharangeal aspirates and swabs, including endocervical, vaginal, occular, throat and buccal swabs; and other biological materials such as finger and toe nails, skin, hair, and cerebrospinal fluid or other body fluid. Environmental samples include soil, water, air, suspension effluents, powders and other sources of nucleic acid containing material.

The biological samples of the present invention may be pretreated to ensure release of nucleic acids into the biological sample for extraction. The pretreatment of biological samples for this purpose are described in U.S. Pre-Grant Publication 2004-0157218 ("'7218"). As disclosed in '7218, a protein denaturant may preferably be used in the pretreatment process. A protein denaturant that is useful in the present invention includes an agent(s) that causes an increase in pH, such as potassium hydroxide (KOH).

The nucleic acids of the present invention are preferably reversibly bound to paramagnetic particles as disclosed by the method of '138 and '160. In '138 and '160, it was found that when in an acidic environment, the paramagnetic particles of the invention will reversibly bind nucleic acid molecules without the necessity of an anionic detergent as taught in International Publication No. WO 96/18731. As used herein, the term paramagnetic particle(s) means particle(s) as described in '138 and '160.

Within the meaning of the present invention, the method steps for separation of the paramagnetic particle-bound nucleic acids from other biological sample components are preferably those method steps disclosed in '138 and '160.

In a preferred embodiment, the paramagnetic particle-bound nucleic acid molecules may be eluted with an appropriate elution buffer accomplished by raising the pH of such environment. In previous methods, the elution step comprised the addition of a buffer designed in general to remove the nucleic acids from the paramagnetic particles and to neutralize the solution at the same time for further manipulation, such as hybridization, restriction, labeling, reverse transcription and amplification. Removing the nucleic acids from the paramagnetic particles in a separate step from neutralization allows optimization of the elution buffer pH for the removal of the nucleic acid, thereby unexpectedly achieving an increased capability to separate and recover unbound nucleic acid relative to that achieved with the previous one-step elution/neutralization type buffers. As described herein, paramagnetic particles, such as iron oxide, bind negatively charged nucleic acids at acidic pH with a net positive charge. At neutral to basic pH, the paramagnetic particles, such as iron oxide, are no longer positively charged and release the nucleic acids. Agents which are used to aid the elution of nucleic acid from paramagnetic particles are basic solutions consisting of potassium hydroxide (KOH), or sodium hydroxide (NaOH) which will increase the pH of the environment to an extent sufficient that electronegative nucleic acid is displaced from the paramagnetic particles.

The condition for elution of nucleic acid occurs at pH values at 8 to 14. Elution at the highest possible pH without degradation is desired to prevent non-specific self-annealing of the nucleic acid strand and to optimize release of the nucleic acids from the paramagnetic particles. Elution at high pH and denaturation of DNA:DNA, DNA:RNA or RNA:RNA hybrids is also beneficial for downstream applications that require single-stranded target, such as hybridization, in particular probe hybridization, or amplification, in particular isothermal nucleic acid amplification. Maintenance of the target nucleic acid in a single-stranded form precludes the need for subsequent heat denaturation prior to hybridization of complementary primers or probes. Self-annealing could promote entanglement of the nucleic acid with the paramagnetic particle itself and prevent separation of the nucleic acid from the paramagnetic particle at the elution step. Other particle types could use the concept of elution followed by neutralization.

The particle-bound nucleic acids are eluted with the elution buffer until the desired result is achieved. For example, the nucleic acids may be eluted from the paramagnetic particles with the addition of an elution buffer composed of KOH and mixing, for example by aspirating and dispensing a given volume, until the desired result is achieved. While this method is successful for separation of DNA and RNA, care should be taken to avoid pH values and/or exposure times that might lead to degradation of nucleic acid.

By removing the bound nucleic acids in this manner, the pH is optimized to achieve the maximum release of bound nucleic acids. Surprisingly, it was found that by performing the elution step separately and allowing for the use of higher pH values resulted in an increased reproducibility of signal generation in downstream nucleic acid amplification assays relative to that achieved using a combined elution/neutralization buffer. The improved capability to recover and/or detect the nucleic acids was unexpected. Therefore, separating the elution step from the neutralization step provides a significant advantage over the previous approaches.

According to the invention, a neutralization buffer is added after the elution step. The neutralization buffer adjusts the pH value of the elution solution containing the unbound nucleic acids to a preferred pH range of 6 to 9, depending on the downstream application, more preferably 8 to 8.5, and most preferable 8.4. By neutralizing the solution containing the unbound nucleic acids in this manner, the pH environment is optimized for further nucleic acid manipulation, such as hybridization, restriction, labeling, reverse transcription and amplification. This may be achieved by using any neutralization buffer suitable for achieving the optimized pH value for further manipulation. A preferred neutralizing buffer is bicine, as is used in the examples below. Alternative neutralization buffers include but are not limited to Tris, CHES [2-(cyclohexylamino)ethanesulfonic acid], BES N,N-Bis(2-hydroxyethyl)-2-aminoethanesulfonic acid], MOPS (4-morpholinepropanesulfonic acid) and phosphate. Other neutralizing buffers useful in the method of the present invention can be readily ascertained by one of skill in the art using routine screening methods that do not require undue experimentation.

After neutralization of the sample, the paramagnetic particles are removed while the pH optimized solution containing the unbound nucleic acids is transferred for further manipulation, such as hybridization, restriction, labeling, reverse transcription and amplification for example. Magnetic force is preferably used to separate the paramagnetic particles, as described herein.

Yet another aspect of the present disclosure is to provide kits for treating a biological sample for the extraction of biological materials there from. The kits may comprise at least one protein denaturant as described herein. The kits may contain water and buffer solutions as described herein, as well as paramagnetic particles or other solid supports for extraction and/or purification, which are described in more detail elsewhere. The kits may also contain one or more of the following items for processing and assaying the biological samples: collection devices such as swabs, tubes and pipettes; controls; pH indicators; and thermometers. Kits may include containers of reagents mixed together in suitable proportions for performing the method in accordance with the present invention. Reagent containers preferably contain reagents in unit quantities that obviate measuring steps when performing the subject method. Kits of the present disclosure may include optimized elution buffers for releasing nucleic acids from paramagnetic particles, as described herein. Kits may include neutralizing buffers for optimizing downstream applications, such as nucleic acid hybridization, restriction, labeling, reverse transcription and amplification, as described herein.

The kits of the present disclosure may also include the reaction mixtures, as well as methods of extracting nucleic acid from the reaction mixtures. The reaction mixtures may comprise at least one protein denaturant for particular embodiments as needed. The reaction mixtures may in some embodiments include various reagents used with the subject reaction mixtures to purify and detect nucleic acids, such as buffers and iron oxide or other solid supports for nucleic acid purification.

### Examples

The invention will now be described in greater detail by way of the specific examples. The following examples are offered for illustrative purposes. The following examples illustrate the effectiveness of the compositions and methods of the present invention to pretreat whole blood and plasma samples for optimized nucleic acid extraction and optimized manipulation. Whole blood and plasma are among the most challenging samples for nucleic acid extraction because of their highly proteinaceous content; therefore, the methods of the present invention are expected to be effective for other biological samples as well. In these examples, the reversible binding of nucleic acid molecules on paramagnetic particles in an acidic environment is used for nucleic acid isolation from the reaction mixture resulting from treating samples for extraction of intact nucleic acid according to the present invention. The binding pH is preferably 1 to 6.5, more preferably 1 to 4, and most preferably 2. The elution pH is preferably 8 to 14. Once of skill in the art will appreciate that the elution pH is preferably optimized by using a pH that is as high as possible without causing degradation of the nucleic acids of the sample. The paramagnetic particle technology captures nucleic acids non-specifically, or independent of sequence. After neutralization, the pH is preferably 6.0-9.0 depending on the downstream application. More preferably the pH is 8 to 8.5, and most preferably 8.4.

### Example 1: Alkali Treatment Elutes DNA From Iron Oxide Better Than Heat Alone

This example was performed to determine if treatment of the samples with 150 mM KOH elutes DNA from the iron oxide better than heat alone.
The materials used in this example were as follows:
300 mM Bicine 2X buffer
Sample buffer
*Chlamydia* Primer wells
*Chlamydia* Amplification wells
Amplification Control (AC) Primer wells
AC Amplification wells
KOH 150 mM
Plasma Samples
Iron oxide
Plasma Pretreatment Tubes (PPT)

Plasma was prepared from whole blood by spinning whole blood in Plasma Pretreatment Tubes (PPT) at 1,100 g for 10 minutes. A 6 ml volume of pooled plasma was prepared. Ten thousand *Chlamydia trachomatis* (CT) Elementary bodies (EB) were added per milliliter to the plasma pool, which was dispensed in equal volumes into six 2 ml centrifuge tubes. Another 10 ml bacterial suspension was prepared in deionized water with 10,000 CT EB/ml and dispensed in 10 x 1 ml volumes. A further suspension was prepared containing 10,000 CT EB/ml in 300 mM Bicine-containing 2X sample buffer.

Forty milligrams of iron oxide were dispensed into four of the tubes of plasma; 80 µl of acetic acid was dispensed into two of the tubes, and 300 µl of acetic acid were added to two tubes containing plasma but no iron oxide. All six of the tubes were placed into a lysolyzer for 30 minutes at 105°C. Forty milligrams of iron oxide were added to the two tubes containing no iron oxide following lysolyzation; 80 µl of acetic acid were added to the two tubes containing no acid. After mixing, recovery of the iron oxide and removal of the specimen matrix, the particles were washed two times with 1 ml/tube of deionized water. One tube of each condition was treated with 500 µl of 150 mM KOH for 15 minutes prior to addition of 300 mM Bicine 2X sample buffer. As controls, one tube from each condition had 75 mM KOH/150 mM Bicine-containing 2X sample buffer added.

Forty milligrams of iron oxide were spiked into two of the 10 tubes with 10,000 CT EB/ml in deionized water. Two tubes containing no iron oxide had 80 µl of acetic acid added and two tubes containing iron oxide had 300 µl of acetic acid added. These tubes and four tubes with no prior acid treatment were lysolyzed at 105°C for 30 minutes. The tubes containing iron oxide prior to lysis had 80 µl dispensed into each. The remaining tubes had 40 mg of iron oxide added and all the tubes were placed on an end-over-end rocker for 30 minutes. After recovery of the iron oxide, the particles were washed two times with 1 ml/tube of deionized water. One tube from each condition was treated with 500 µl of 150 mM KOH for 15 minutes prior to addition of 300 mM Bicine 2X sample buffer. As controls, one tube of each type had 75 mM KOH/150 mM Bicine 2X sample buffer added.

The eluates from all the tubes were boiled for 5 minutes and the lysates were tested using microwells from the BD ProbeTec™ *Chlamydia trachomatis* Amplified DNA Assay (Little et al, Clin Chem 1999; 45:777-784).

**Table 1**

| SAMPLE | Ferric Oxide in LYSOLYZER | Acetic Acid in LYSOLYZER | ALKALI TREATMENT | CT MOTA | AC MOTA* |
|---|---|---|---|---|---|
| Plasma | YES | NO 80 µl | YES | 12988 | 9978 |
| Plasma | YES | NO 80 µl | NO | 3937 | 18449 |
| | | | | | |
| Clean | YES | NO 80 µl | YES | 13664 | 9869 |
| Clean | YES | NO 80 µl | NO | 116 | 5129 |
| | | | | | |
| Clean | NO | NO 80 µl | YES | 11727 | 8207 |
| Clean | NO | NO 80 µl | NO | 234 | 10788 |
| | | | | | |
| Plasma | YES | YES 80 µl | YES | 84 | 4014 |
| Plasma | YES | YES 80 µl | NO | 158 | 10916 |
| | | | | | |
| Clean | NO | YES 80 µl | YES | 160 | 7765 |
| Clean | NO | YES 80 µl | NO | 194 | 8481 |
| | | | | | |
| Plasma | NO | YES 300 µl | YES | 77 | 9817 |
| Plasma | NO | YES 300 µl | NO | 244 | 3541 |
| | | | | | |
| Clean | NO | YES 300 µl | YES | 5 | 4670 |
| Clean | NO | YES 300 µl | NO | 97 | 1931 |
| | | | | | |
| Clean | NO | NO 300 µl | YES | 1360 | 42 |
| Clean | NO | NO 300 µl | NO | 176 | 610 |
| | | | | | |
| SB Control | Sample Buffer | | | 33912 | 12048 |
| SB Control | Sample Buffer | | | 23450 | 9601 |

| | | | | | |
|---|---|---|---|---|---|
| * AC - Amplification Control | | | | | |

The MOTA (Metric Other Than Acceleration) value represents the area under the curve of relative fluorescence over time. The established cutoff for a positive reaction with the CT assay is 2,000 MOTA. It is evident that, in the majority of cases, higher MOTA scores were obtained from lysates exposed to the two-step elution process (KOH followed by neutralization with Bicine).

### Example 2: Smaller Elution Volume Used With Two Step Elution

This example demonstrates recovery of RNA using a two-step elution process.
The materials used in this example were as follows:
Ferric Oxide
Plasma Pretreatment Tubes (PPT)
30 mM KP04
500 mM KP04
Avian Myeloblastosis Virus Reverse Transcriptase (AMV-RT)
BsoBI restriction enzyme
GP32 protein
Bovine Serum Albumin (BSA)
Bst polymerase
55% Glycerol
200mM Magnesium
Dimethylsulfoxide (DMSO)
Fluorescent Detector Probe
Strand Displacement Amplification (SDA) primers
Bumper Primers
Deoxyribonucleotide triphosphates (dNTPs)
Proteinase K
Formamide
Binding Acid
KOH
Bicine
HIV *gag* gene transcripts

Plasma was pretreated with 44% formamide and 5U Proteinase K for 20 minutes at 65 °C and 10 minutes at 70 °C. Iron oxide and 180 µl of binding acid were added to the plasma. The mixtures were then spiked at 10,000 copies/ml of HIV *gag* gene transcript. After binding to the ferric oxide and washing, the RNA was eluted with 120 µl of either 80 mM or 100 mM KOH elution buffer for 20 minutes at 65 °C. The remaining eluate was neutralized with 60 µl of either 192 mM or 230 mM bicine, and mixed for 2 minutes. The RNA was reverse transcribed with AMV-RT and amplified by SDA using gag-specific primers. (Nycz et al., Anal Biochem, 1998; 259:226-234). Detection occurred in real time using a fluorescent detector probe. (Nadeau et al., Anal Biochem, 1999; 276:177-187).

**Table 2**

| KOH ELUTION (mM) | BICINE NEUTRALIZATION (mM) | HIV TRANSCRIPT CONCENTRATION/ML | HIV/MOTA | MEAN |
|---|---|---|---|---|
| 80 | 230 | 4000 | 4108 | |
| 80 | 230 | 4000 | 2098 | |
| 80 | 230 | 4000 | 6550 | 4252 |
| | | | | |
| 80 | 230 | 8000 | 37915 | |
| 80 | 230 | 8000 | 1501 | |
| 80 | 230 | 8000 | 9832 | 16416 |
| | | | | |
| 80 | 192 | 4000 | 2 | |
| 80 | 192 | 4000 | 13 | |
| 80 | 192 | 4000 | 863 | 299 |
| | | | | |
| 80 | 192 | 8000 | 24648 | |
| 80 | 192 | 8000 | 24957 | |
| 80 | 192 | 8000 | 41701 | 30435 |
| | | | | |
| 100 | 230 | 4000 | 0 | |
| 100 | 230 | 4000 | 0 | |
| 100 | 230 | 4000 | 6 | 3 |
| | | | | |
| 100 | 230 | 8000 | 0 | |
| 100 | 230 | 8000 | 4 | |
| 100 | 230 | 8000 | 1 | 2 |
| | | | | |
| 100 | 192 | 4000 | 0 | |
| 100 | 192 | 4000 | 0 | |
| 100 | 192 | 4000 | 0 | 0 |
| | | | | |
| 100 | 192 | 8000 | 0 | |
| 100 | 192 | 8000 | 0 | |
| 100 | 192 | 8000 | 3 | 1 |

The samples for which the lower 80mM KOH concentration was used for elution produced higher MOTA values, indicating more robust amplification/detection of target RNA. It is likely that exposure to the higher concentration of KOH (100mM) caused hydrolysis and degradation of the RNA transcripts. This experiment therefore demonstrates the ability of ferric oxide extraction with the two step elution process to recover RNA from a complex biological matrix. Unexpectedly, exposure of RNA to a high pH during the elution step did not cause degradation of the target nucleic acid.

### EXAMPLE 3: Effect of Heat During Two Step Elution

The example was performed to determine if heat during elution at different KOH concentrations affects the stability and/or recovery and/amplification/detection of RNA.
The materials used in this example were as follows:
Ferric Oxide
Plasma Preparation Tubes (PPT)
30 mM KP04
500 mM KP04
AMV RT
BsoBI Restriction enzyme
GP32 protein
Bovine Serum Albumin (BSA)
Bst polymerase
55% Glycerol
200mM Magnesium
Dimethylsulfoxide (DMSO)
Fluorescent Detector Probe
Strand Displacement Amplification (SDA) primers
Bumper Primers
Deoxyribonucleotide triphospates (dNTPs)
Proteinase K
Formamide
Binding Acid
KOH
Bicine
HIV *gag* gene transcripts

Plasma was pretreated with 44% formamide and 5U Proteinase K for 20 minutes at 65 °C and 10 minutes at 70 °C.Iron oxide and 180 µl of binding acid were added to the plasma. The mixtures were then spiked at 5,000 copies of HIV *gag* gene transcript/ml. After binding to the ferric oxide and washing, the RNA was eluted with 120 µl of either 60 mM, 70mM or 80mM KOH elution buffer for either 2 minutes without heat or for 20 minutes at 65 °C. The samples were neutralized immediately by mixing with 60 µl of 230 mM bicine for 2 minutes. The RNA was reverse transcribed with AMV-RT and amplified by SDA using gag-specific primers. (Nycz et al., Anal Biochem, 1998; 259:226-234). Detection occurred in real time using a fluorescent detector probe. (Nadeau et al., Anal Biochem, 1999; 276:177-187).

**Table 3**

| ELUTION KOH (mM) | | MOTA | MEAN |
|---|---|---|---|
| 60 | NO HEAT | 20256 | |
| 60 | NO HEAT | 14841 | |
| 60 | NO HEAT | 13690 | |
| 60 | NO HEAT | 3821 | 13152 |
| | | | |
| 70 | NO HEAT | 23759 | |
| 70 | NO HEAT | 5870 | |
| 70 | NO HEAT | 1923 | |
| 70 | NO HEAT | 11908 | 10865 |
| | | | |
| 80 | NO HEAT | 6006 | |
| 80 | NO HEAT | 21826 | |
| 80 | NO HEAT | 4887 | |
| 80 | NO HEAT | 17973 | 12623 |
| | | | |
| 60 | HEAT | 34805 | |
| 60 | HEAT | 25907 | |
| 60 | HEAT | 18274 | |
| 60 | HEAT | 6884 | 21467 |
| | | | |
| 70 | HEAT | 14220 | |
| 70 | HEAT | 18591 | |
| 70 | HEAT | 3872 | |
| 70 | HEAT | 2297 | 9745 |
| 80 | HEAT | 3220 | |
| 80 | HEAT | 3930 | |
| 80 | HEAT | 75 | |
| 80 | HEAT | 0 | 1806 |

Positive MOTA values (>2000) were obtained under all conditions. These data, therefore, indicate that it may be possible to elute RNA from ferric oxide without employing heat using a two-step elution method involving exposure to KOH followed by neutralization with bicine. The procedure without heat has the advantage of requiring less sophisticated instrumentation.

### EXAMPLE 4: Optimization of Elution Conditions

This experiment was performed to optimize elution conditions. The materials used in this example were as follows:
Ferric Oxide
Plasma Preparation Tubes (PPT)
30 mM KP04
500 mM KP04
AMV RT
BsoBI Restriction enzyme
GP32 protein
Bovine Serum Albumin (BSA)
Bst polymerase
55% Glycerol
200mM Magnesium
Dimethylsulfoxide (DMSO)
Fluorescent Detector Probe
Strand Displacement Amplification (SDA) primers
Bumper Primers
Deoxyribonucleotide triphospates (dNTPs)
Proteinase K
Formamide
Binding Acid
KOH
Bicine
HIV *gag* gene transcripts

Plasma was pretreated with 44% formamide and 5U Proteinase K for 20 minutes at 65 °C and 10 minutes at 70 °C. Iron oxide and 180 µl of binding acid were added to the plasma. The mixtures were then spiked at 10,000 copies of HIV *gag* gene transcript/ml. After binding to the ferric oxide and washing, the RNA was eluted with 120 µl of either 46mM, 55mM, 63 mM or 80 mM KOH elution buffer for 20 minutes at 65 °C. The samples were then neutralized with 60 µl of 109 mM bicine and mixed for 2 minutes. The RNA was reverse transcribed with AMV-RT and amplified by SDA using gag-specific primers. (Nycz et al., Anal Biochem, 1998; 259:226-234). Detection occurred in real time using a fluorescent detector probe. (Nadeau et al, Anal Biochem, 1999; 276:177-187).

**Table 4**

| CONDITION | | MOTA | MEAN |
|---|---|---|---|
| 80 mM KOH, 109 mM bicine, 24 mM KP04 | HEAT | 5887 | |
| 80 mM KOH, 109 mM bicine, 24 mM KP04 | HEAT | 5648 | |
| 80 mM KOH, 109 mM bicine, 24 mM KP04 | HEAT | 7377 | 6304 |
| | | | |
| 63 mM KOH, 109 Mm bicine, 50 mM KP04 | HEAT | 5339 | |
| 63 mM KOH, 109 Mm bicine, 50 mM KP04 | HEAT | 4586 | |
| 63 mM KOH, 109 Mm bicine, 50 mM KP04 | HEAT | 1648 | 3857 |
| | | | |
| 46 mM KOH, 46 mM bicine, 36 mM KP04 | HEAT | 4731 | |
| 46 mM KOH, 46 mM bicine, 36 mM KP04 | HEAT | 6466 | |
| 46 mM KOH, 46 mM bicine, 36 mM KP04 | HEAT | 6147 | 5781 |
| | | | |
| 55 mM KOH, 56 mM bicine, 43 mM KP04 | HEAT | 5656 | |
| 55 mM KOH, 56 mM bicine, 43 mM KP04 | HEAT | 10620 | |
| 55 mM KOH, 56 mM bicine, 43 mM KP04 | HEAT | 9606 | 8627 |
| | | | |
| 80 mM KOH, 109 mM bicine, 24 mM KP04 | NO HEAT | 5430 | |
| 80 mM KOH, 109 mM bicine, 24 mM KP04 | NO HEAT | 3559 | |
| 80 mM KOH, 109 mM bicine, 24 mM KP04 | NO HEAT | 1566 | 3518 |
| | | | |
| 63 mM KOH, 109 mM bicine, 50 mM KP04 | NO HEAT | 72 | |
| 63 mM KOH, 109 mM bicine, 50 mM KP04 | NO HEAT | 91 | |
| 63 mM KOH, 109 mM bicine, 50 mM KP04 | NO HEAT | 107 | 90 |
| | | | |
| 46 mM KOH, 46 mM bicine, 36 mM KP04 | NO HEAT | 2087 | |
| 46 mM KOH, 46 mM bicine, 36 mM KP04 | NO HEAT | 2581 | |
| 46 mM KOH, 46 mM bicine, 36 mM KP04 | NO HEAT | 2004 | 2224 |
| | | | |
| 55 mM KOH, 56 mM bicine, 43 mM KP04 | NO HEAT | 1122 | |
| 55 mM KOH, 56 mM bicine, 43 mM KP04 | NO HEAT | 1608 | |
| 55 mM KOH, 56 mM bicine, 43 mM KP04 | NO HEAT | 2782 | 1838 |

RNA was successfully recovered from plasma using the two step elution procedure. These data show, however, that higher MOTA values were obtained when the RNA was eluted in the presence of heat, irrespective of the buffer conditions employed for amplification/detection.

### EXAMPLE 5: Smaller Elution Volume with Two Step Elution

The example evaluated smaller elution volume with the two-step elution process.
The materials used in this example were as follows:
Ferric Oxide
Plasma Preparation Tubes (PPT)
30 mM KP04
500 mM KP04
AMV RT
BsoBI Restriction enzyme
GP32 protein
Bovine Serum Albumin (BSA)
Bst polymerase
55% Glycerol
200mM Magnesium
Dimethylsulfoxide (DMSO)
Fluorescent Detector Probe
Strand Displacement Amplification (SDA) primers
Bumper Primers
Deoxyribonucleotide triphospates (dNTPs)
Proteinase K
Formamide
Binding Acid
KOH
Bicine
HIV *gag* gene transcripts

Plasma was pretreated with 44% formamide and 5U Proteinase K for 20 minutes at 65 °C and 10 minutes at 70 °C.Iron oxide and 180 µl of binding acid were added to the plasma. The mixtures were then spiked at 10,000 copies of HIV *gag* gene transcript/ml. After binding to the ferric oxide and washing, the RNA was eluted with 120 µl of either 50 mM, 65 mM, and 80 mM KOH for 20 minutes at 65 °C. The samples were then neutralized with 60 µl of either 154 mM, 192 mM or 230 mM bicine and mixed for two minutes. The RNA was reverse transcribed with AMV-RT and amplified by SDA using gag-specific primers. (Nycz et al., Anal Biochem, 1998; 259:226-234). Detection occurred in real time using a fluorescent detector probe. (Nadeau et al., Anal Biochem, 1999; 276:177-187).

**Table 5**

| ELUTION KOH (mM). | NEUTRALIZATION BICINE (mM) | FINAL KOH | FINAL BICINE | MOTA | MEAN |
|---|---|---|---|---|---|
| 80 | 230 | 42 | 86 | 74494 | |
| 80 | 230 | 42 | 86 | 73007 | |
| 80 | 230 | 42 | 86 | 59702 | 69068 |
| | | | | | |
| 80 | 192 | 42 | 76 | 59816 | |
| 80 | 192 | 42 | 76 | 67597 | |
| 80 | 192 | 42 | 76 | 70179 | 65864 |
| | | | | | |
| 80 | 154 | 42 | 66 | 64613 | |
| 80 | 154 | 42 | 66 | 62096 | |
| 80 | 154 | 42 | 66 | 64866 | 53858 |
| | | | | | |
| 65 | 192 | 34 | 76 | 72410 | |
| 65 | 192 | 34 | 76 | 87738 | 70074 |
| | | | | | |
| 65 | 154 | 34 | 86 | 57300 | |
| 65 | 154 | 34 | 86 | 37732 | 47516 |
| | | | | | |
| 50 | 230 | 26 | 86 | 65206 | |
| 50 | 230 | 26 | 86 | 30787 | 47997 |
| | | | | | |
| 50 | 192 | 26 | 76 | 68328 | |
| 50 | 192 | 26 | 76 | 54644 | 81486 |
| | | | | | |
| 50 | 154 | 26 | 66 | 60811 | |
| 50 | 154 | 26 | 66 | 57274 | 59043 |
| | | | | | |
| 50 | control | 50 | 90 | 58761 | |
| 50 | control | 50 | 90 | 65975 | 62358 |

Robust amplification of the RNA target was achieved under each of the conditions tested, as determined by the high MOTA scores. These data demonstrate the utility of iron oxide extraction followed by a two-step elution process for the recovery of amplifiable RNA from a complex biological matrix. No RNA hydrolysis was evident from exposure to different concentrations of KOH for 20 min at 65 °C.

### EXAMPLE 6: Two Step Elution and Neutralization

This example details the separation of elution and neutralization steps compared to one-step method and the effect on MOTA.
The materials used in this example were as follows:
Ferric Oxide
Plasma Preparation Tubes (PPT)
30 mM KP04
500 mM KP04
AMV RT
BsoBI Restriction enzyme
GP32 protein
Bovine Serum Albumin (BSA)
Bst polymerase
55% Glycerol
200mM Magnesium
Dimethylsulfoxide (DMSO)
Fluorescent Detector Probe
Strand Displacement Amplification (SDA) primers
Bumper Primers
Deoxyribonucleotide triphospates (dNTPs)
Proteinase K
Formamide
Binding Acid
KOH
Bicine
HIV *gag* gene transcripts

Plasma was pretreated with 44% formamide and 5U Proteinase K for 20 minutes at 65 °C and 10 minutes at 70 °C. Iron oxide and 180 µl of binding acid were added to the plasma. The mixtures were then spiked at 10,000 copies of HIV *gag* gene transcript/ml. After binding to the ferric oxide and washing, the RNA was eluted with 400 µl of either 50 mM, 65 mM or 80 mM KOH elution buffer for 20 minutes at 65 °C. The eluates were split into volumes of 100 µl and 300 µl each of which was neutralized with a different bicine-containing neutralization buffer (Table 6). The RNA was reverse transcribed with AMV-RT and amplified by SDA using gag-specific primers. (Nycz et al., Anal Biochem, 1998; 259:226-234). Detection occurred in real time using a fluorescent detector probe. (Nadeau et al., Anal Biochem, 1999; 276:177-187).

**Table 6**

| ELUTION KOH (mM) | NEUTRALIZATION BICINE (mM) | MOTA | MEAN MOTA | | FINAL KOH (mM) | FINAL BICINE (mM) |
|---|---|---|---|---|---|---|
| 80 | 0/160 | 49050 | | | 40 | 110 |
| 80 | 0/160 | 45345 | | | 40 | 110 |
| 80 | 0/160 | 34158 | 42851 | | 40 | 110 |
| | | | | | | |
| 80 | 0/130 | 36091 | | | 40 | 90 |
| 80 | 0/130 | 39036 | | | 40 | 90 |
| 80 | 0/130 | 46476 | 40534 | | 40 | 90 |
| | | | | | | |
| 80 | 0/100 | 64709 | | | 40 | 75 |
| 80 | 0/100 | 65277 | | | 40 | 75 |
| 80 | 0/100 | 40217 | 50058 | | 40 | 75 |
| | | | | | | |
| 65 | 0/160 | 54037 | | | 32.5 | 110 |
| 65 | 0/160 | 60464 | | | 32.5 | 110 |
| 65 | 0/160 | 56883 | 57061 | | 32.5 | 110 |
| | | | | | | |
| 65 | 0/130 | 56187 | | | 32.5 | 90 |
| 65 | 0/130 | 55621 | 65904 | | 32.5 | 90 |
| | | | | | | |
| 65 | 0/100 | 52745 | | | 32.5 | 75 |
| 65 | 0/100 | 54458 | 53602 | | 32.5 | 75 |
| | | | | | | |
| 50 | 0/160 | 70757 | | | 25 | 110 |
| 50 | 0/160 | 60795 | 65776 | | 25 | 110 |
| | | | | | | |
| 50 | 0/130 | 72728 | | | 25 | 90 |
| 50 | 0/130 | 67532 | 70130 | | 25 | 90 |
| | | | | | | |
| 50 | 0/100 | 69772 | | | 25 | 75 |
| 50 | 0/100 | 66012 | 67892 | | 25 | 75 |
| | | | | | | |
| ONE STEP | CONTROL | 84066 | | | 50 | 90 |
| ONE STEP | CONTROL | 69863 | 71965 | | 50 | 90 |
| | | | | | | |
| 80 | 20/160 | 34865 | | | 50 | 110 |
| 80 | 20/160 | 6098 | | | 50 | 110 |
| 80 | 20/160 | 2670 | 14544 | | 50 | 110 |
| | | | | | | |
| 80 | 20/130 | 34874 | | AMPLIFICATION CONTROL | 50 | 90 |
| 80 | 20/130 | 8710 | | | 50 | 90 |
| 80 | 20/130 | 29190 | 24258 | | 50 | 90 |
| | | | | | | |
| 80 | 20/100 | 47498 | | | 50 | 75 |
| 80 | 20/100 | 20794 | | | 50 | 75 |
| 80 | 20/100 | 44890 | 37727 | | 50 | 75 |
| 65 | 35/160 | 45072 | | | 50 | 110 |
| 65 | 35/160 | 50814 | | | 50 | 110 |
| 65 | 35/160 | 41113 | 45686 | | 50 | 110 |
| | | | | | | |
| 65 | 35/130 | 33511 | | AMPLIFICATION CONTROL | 50 | 90 |
| 65 | 35/130 | 22663 | 28087 | | 50 | 90 |
| | | | | | | |
| 65 | 35/100 | 64496 | | | 50 | 75 |
| 65 | 35/100 | 68245 | 61370 | | 50 | 75 |
| | | | | | | |
| 50 | 50/160 | 6536 | | | 50 | 110 |
| 50 | 50/160 | 14936 | 10736 | | 50 | 110 |
| | | | | | | |
| 50 | 50/130 | 55468 | | AMPLIFICATION CONTROL | 50 | 90 |
| 50 | 50/130 | 15955 | 35711 | | 50 | 90 |
| | | | | | | |
| 50 | 50/100 | 44669 | | | 50 | 75 |
| 50 | 50/100 | 56643 | 55656 | | 50 | 75 |
| | | | | | | |
| ONE STEP | CONTROL | 70602 | | | CONTROL | CONTROL |
| ONE STEP | CONTROL | 76028 | 73315 | | CONTROL | CONTROL |

MOTA scores improved with decreased KOH concentration during elution, suggesting that the RNA target might be partially degraded by prolonged exposure to strong alkali. Elution with lower concentration KOH improved MOTA scores indicating more robust amplification/detection.

### EXAMPLE 7: Elution Efficiency With Target DNA

The purpose of this experiment was to determine the elution efficiency of DNA from ferric oxide using the BD Viper™ System in extracted mode. This study was designed to evaluate whether there was amplifiable target DNA still bound to the iron oxide after the final elution step in the ferric oxide extraction process when conducted using an SDA compatible buffer (approximately pH 8.4). In a previous experiment it was determined that if ferric oxide is re-exposed to elution buffer of this pH and the second eluate tested in an SDA reaction positive fluorescent signals will result. One of the possible reasons for this was to the presence of trace quantities of elution buffer after the original extraction. To mitigate this potential, all extraction tubes in this experiment had the remaining elution buffer form the initial extraction event removed prior to re-elution with additional SDA compatible buffer. This was accomplished by washing the ferric oxide with deionized water (pH 4-5) to prevent further elution of any bound DNA. No clinical matrix was used in this experiment.
The materials used in this example were as follows:
Potassium phosphate-DMSO-glycerol (KPDG) Sample Diluent (SDA compatible buffer)
Extraction Tubes
Lysis Buffer
Binding Buffer
Wash Buffer
Elution Buffer
Priming and Amplification Microwells for the BD ProbeTec™ CT/GC Q^{x} Amplified DNA Assays *Chlamydia trachomatis* (CT)/*Neisseria gonnorhea* (GC) organisms (1x10⁵ /mL stock)
The procedure was as follows:

| | |
|---|---|
| 1 | Viper SP instruments (PP001 - V3.00H+) were used for the testing. |
| 2 | Diag switch the NUM_WASH_MIXES=2, and ELUT_VOL_400, NO_LIQUID=1 |
| 3 | Rebooted each instrument with the appropriate Diagnostic disk. |
| 4 | Prepared 70mL of 50 each organism/mL (CT and GC) by adding 35µL of 10⁵/mL CT/GC stock into 70mL of CT/GC sample diluent. |
| 5 | Aliquotted 1 mL of the positive diluent into 48 sample diluent tubes. |
| 6 | Set up the Viper instrument for a half extraction run with CTQ^{X}/GCQ^{X} plates. |
| 7 | PP001, Rack #14 - primary control extraction run. |
| 8 | After the first run, removed the extraction tubes from the Viper extraction block. |
| 9 | Inserted all tubes into the manual Viper extraction block. |
| 10 | Engaged the magnets to lockdown the iron oxide. |
| 11 | With a Matrix pipettor, removed all remaining potassium phosphate DMSO-glycerol (KPDG) elution buffer fluid from the appropriate extraction tubes. |
| 12 | Disengaged the magnets. |
| 13 | Added 1 mL of DiH2O to 24 used extraction tubes. Mixed. |
| 14 | Engaged the magnets to lockdown the iron oxide. |
| 15 | Removed the wash elutes and dispensed into new sample diluent tubes. |
| 16 | Repeated the process for 12 of the 24 used extraction tubes. |
| 17 | Added the wash eluate specimens to the Viper specimen rack. |
| 18 | Added 2X KPDG elution buffer to each of the wash elutes. |
| 19 | Added all the used extraction tubes back into the Viper extraction rack. |

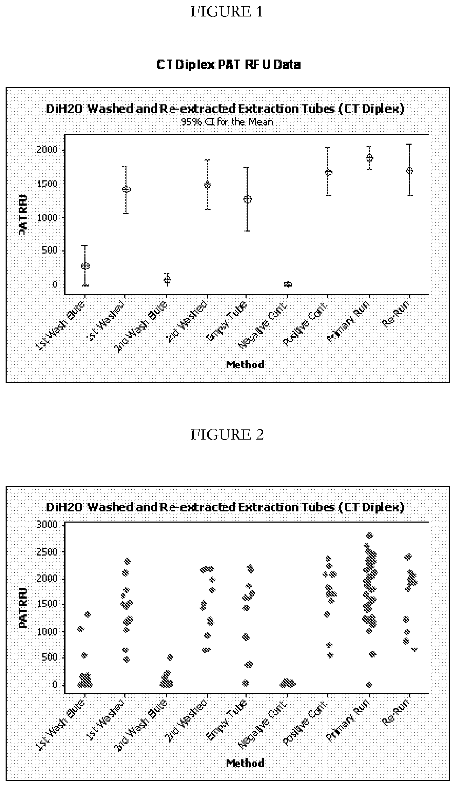

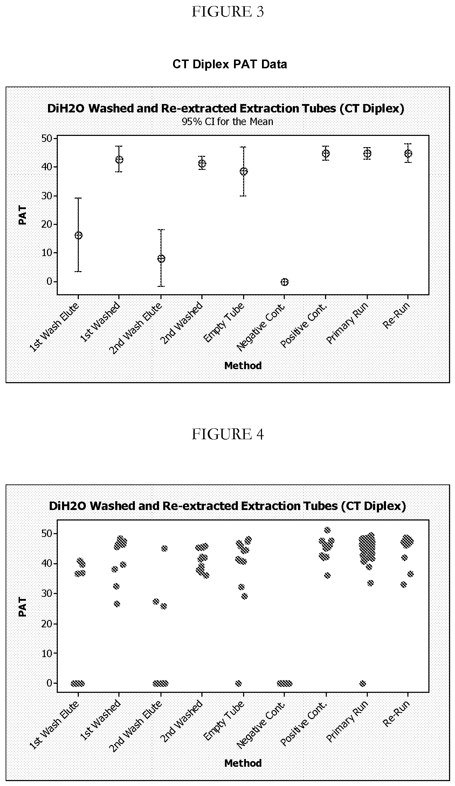

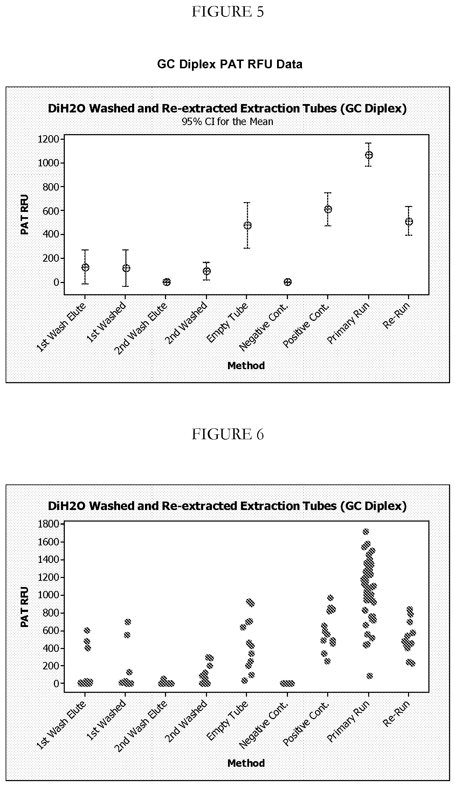

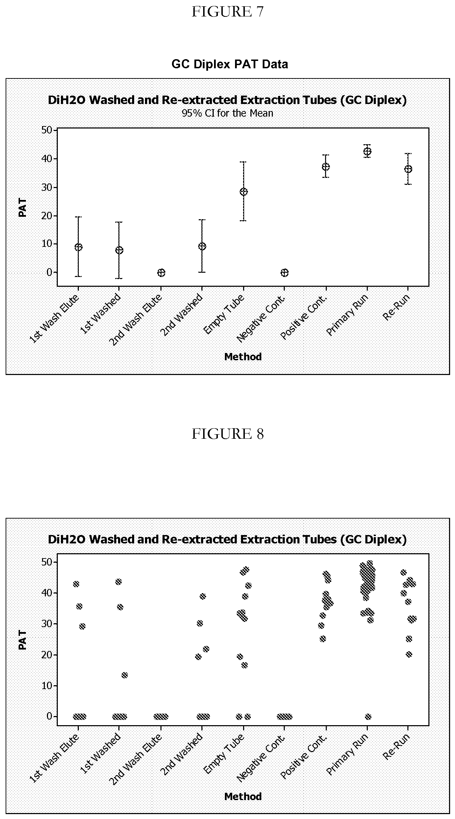

The results, shown in Figures 1 - 8, indicate that there was amplifiable CT/GC target DNA still bound to the iron oxide after the initial elution step with KPDG buffer at approximately pH 8.4. Washing the iron oxide with deionized water removed traces of the first eluate without eluting the remaining target DNA from the iron oxide. Further treatment of the iron oxide with additional KPDG elution buffer allowed recovery of more target DNA that was detectable by SDA. To follow up this experiment a higher pH elution buffer was evaluated to recover the remaining target DNA from the iron oxide. One of skill in the art would have the ability to evaluate various such buffer conditions without undue experimentation.

### EXAMPLE 8: 2-Step Elution MSA

The purpose of this experiment is to complete a Measurement System Analysis for the two-step elution process using the BD Viper™ System in extracted mode to determine the reproducibility of results between runs and Viper instruments.

Two-step elution means the addition of 2X KOH solution (142 mM) to extraction tubes followed by 2X neutralization solution to form the SDA assay buffer (2X neutralization solution is 251mM Bicine, 21.8% DMSO, 19% Glycerol, with 0.1% Tween 20 and 0.03% Proclin 300).
The materials used in this experiment were as follows:
CT/GC Sample Diluent 5.9L
Extraction Tubes 15 trays
2X Neutralization Buffer 250ml
2X KOH (High pH Elution Buffer) 250ml
Wash Buffer (water and Tween)
Binding Acid
KOH lysis buffer
Priming and Amplification Microwells for the BD ProbeTec™ CT/GC Q^{x} Amplified DNA Assays *Chlamydia trachomatis* (CT) 10⁵ spiker 2 aliquots
*Neisseria gonnorhea* (GC) 10⁵ spiker 4 aliquots

CT/GC positive and negative samples were prepared in Sample Diluent. The low target pool was spiked with CT at 15 EB/ml and GC at 50 cells/ml. The high target pool was spiked with CT at 30 EB/ml and GC at 100 cells/ml. The spiking calculations were as follows:
Low: CT 15 EB/ml: 10⁵/ml (xmls) = 15 EB/ml (2450 ml) ==> 367.5 µl CT spike;
GC 50 cells/ml: 10⁵/ml (xmls) = 50 cells/ml (2450 ml) ==> 1225 µl GC spike.
High: CT 30 EB/ml: 10⁵/ml (xmls) = 30 EB/ml (2450mls) ==> 735 µl CT spike;
GC 100 cells/ml: 10⁵/ml (xmls) = 100 cells/ml (2450mls) ==> 2450 µl GC spike.
The CT/GC negative samples were left unspiked. The samples were aliquoted into 5 separate Viper racks at 3.5ml/tube for 3 extraction events from each tube The same samples were used for all three runs on each instrument. Samples were extracted using either a one-step or two-step elution protocol. In brief, KOH was added to the samples to lyse the cells and liberate their nucleic acid into solution. Binding acid was then added to lower the pH and bring about a positive charge on the surface of the ferric oxide, which in turn bound the negatively charged DNA. The ferric oxide and bound DNA were washed and the DNA was eluted either in a two-step process involving exposure to KOH followed by neutralization with bicine buffer, or in a one-step process involving exposure to a solution of bicine and KOH at approximately pH 8.4. The eluted DNA was then detected using the BD ProbeTec™ CT/GC Q^{x} Amplified DNA Assays.

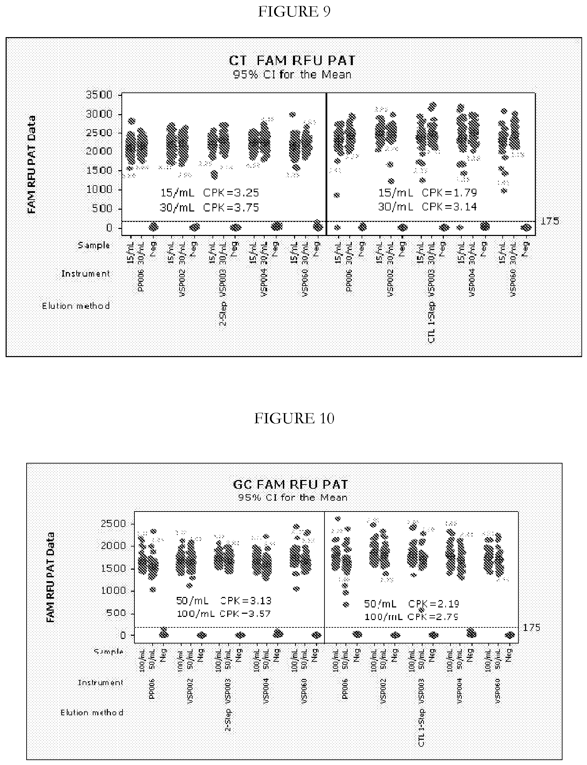

The results are shown in Figures 9-12, which depict the Maximum Relative Fluorescent Units (MaxRFU) obtained with each extracted specimen. A higher MaxRFU is indicative of more efficient amplification/detection. The tighter the clustering of MaxRFU scores, the more robust the system. In Figures 9 and 11, the two-step CT low sample type (15 EB/ml) gave a CpK that was 1.46 higher than that of the one-step elution method. In Figures 10 and 12, the two-step GC low sample type (50 cells/ml) gave a CpK that was 0.94 higher than that of the one-step elution method. The CpK is the capability index, a measure of variation in long term or large samples of data that include not only variation about the mean but also the shifting of the mean itself. CpK is a common metric that is used during steady state production to measure reproducibility of performance.

The two-step elution process performed better and gave significantly higher CpK values than the one-step elution program for both CT and GC.

## Claims

1. A method for extracting nucleic acid components of a biological sample, comprising:
(i) in a container, reversibly binding at least one nucleic acid component of the biological sample to at least one paramagnetic particle, said paramagnetic particle being an iron oxide particle in an acid environment;
(ii) separating the at least one paramagnetic particle bound nucleic acid component from unbound components of the biological sample;
(iii) washing the at least one paramagnetic particle bound nucleic acid component;
(iv) separating the at least one paramagnetic particle bound nucleic acid component from the wash;
(v) removing the at least one nucleic acid component from the at least one paramagnetic particle by a two-step elution and neutralization process comprising
- eluting the at least one paramagnetic particle bound nucleic acid component with a basic pH elution buffer solution consisting of potassium hydroxide or sodium hydroxide, thereby yielding an eluted sample; and
- followed by neutralizing the eluted sample by subsequently adding a neutralizing buffer selected from bicine, Tris, 2-(cyclohexyl-amino)ethanesulfonic acid (CHES), N,N-bis(2-hydroxyethyl)-2-aminoethanesulfonic acid (BES), 4-morpholinepropanesulfonic acid (MOPS) or phosphate, thereby yielding an optimized buffer,
(vi) followed by separating and removing the paramagnetic particles after neutralization of the sample while the pH-optimized solution containing the unbound nucleic acid is transferred for further manipulation,
wherein the removing step is separate from the neutralizing step.

2. The method of claim 1, wherein the biological sample is clinical, forensic or environmental.

3. The method of claim 2, wherein the biological sample is environmental comprising soil, water, air, suspension effluents or powder.

4. The method of claim 1, wherein the biological sample is pretreated to lyse cells.

5. The method of claim 1, wherein said elution comprises raising the pH with the pH elution buffer.

6. The method of claim 1, wherein the elution buffer has a pH of 8 to 14.

7. The method of claim 1, wherein said neutralizing buffer lowers the pH.

8. The method of claim 7, wherein the pH is 6 to 9, preferably wherein the pH is 8 to 8.5, more preferably wherein the pH is 8.4 after the addition of the neutralizing buffer.

## Patentansprüche

1. Verfahren zum Extrahieren von Nucleinsäurekomponenten einer biologischen Probe, umfassend:
(i) reversibles Binden wenigstens einer Nucleinsäurekomponente der biologischen Probe an wenigstens ein paramagnetisches Teilchen in einem Behälter, wobei das paramagnetische Teilchen ein Eisenoxidteilchen in einer sauren Umgebung ist;
(ii) Trennen der an das wenigstens eine paramagnetische Teilchen gebundenen Nucleinsäurekomponente von ungebundenen Komponenten der biologischen Probe;
(iii) Waschen der an das wenigstens eine paramagnetische Teilchen gebundenen Nucleinsäurekomponente;
(iv) Abtrennen der an das wenigstens eine paramagnetische Teilchen gebundenen Nucleinsäurekomponente von der Waschlösung;
(v) Entfernen der wenigstens einen Nucleinsäurekomponente von dem wenigstens einen paramagnetischen Teilchen durch eine ZweiSchritt-Elution und ein Neutralisierungsverfahren, umfassend:
- Eluieren der an das wenigstens eine paramagnetische Teilchen gebundenen Nucleinsäurekomponente mit einer Elutionspufferlösung mit basischem pH-Wert, die aus Kaliumhydroxid oder Natriumhydroxid besteht, wodurch man eine eluierte Probe erhält; und
- anschließendes Neutralisieren der eluierten Probe durch anschließendes Hinzufügen eines neutralisierenden Puffers, der aus Bicin, Tris, 2-(Cyclohexylamino)ethansulfonsäure (CHES), N,N-Bis(2-hydroxyethyl)-2-aminoethansulfonsäure (BES), 4-Morpholinpropansulfonsäure (MOPS) oder Phosphat ausgewählt ist, wodurch man einen optimierten Puffer erhält;
(vi) anschließendes Abtrennen und Entfernen der paramagnetischen Teilchen nach der Neutralisation der Probe, während die pHoptimierte Lösung, die die ungebundene Nucleinsäure enthält, zur weiteren Verarbeitung übertragen wird;
wobei der Entfernungsschritt von dem Neutralisationsschritt getrennt ist.

2. Verfahren gemäß Anspruch 1, wobei die biologische Probe eine klinische, forensische oder Umweltprobe ist.

3. Verfahren gemäß Anspruch 2, wobei die biologische Probe eine Umweltprobe ist, die Erde, Wasser, Luft, Suspensionseffluenten oder Pulver umfasst.

4. Verfahren gemäß Anspruch 1, wobei die biologische Probe vorbehandelt wird, um Zellen zu lysieren.

5. Verfahren gemäß Anspruch 1, wobei die Elution das Anheben des pH-Werts mit dem pH-Elutionspuffer umfasst.

6. Verfahren gemäß Anspruch 1, wobei der Elutionspuffer einen pH-Wert von 8 bis 14 aufweist.

7. Verfahren gemäß Anspruch 1, wobei der neutralisierende Puffer den pH-Wert senkt.

8. Verfahren gemäß Anspruch 7, wobei der pH-Wert 6 bis 9 beträgt, wobei vorzugsweise der pH-Wert 8 bis 8,5 beträgt, wobei besonders bevorzugt der pH-Wert nach der Zugabe des neutralisierenden Puffers 8,4 beträgt.

## Revendications

1. Procédé d'extraction de composants d'acide nucléique d'un échantillon biologique, comprenant :
(i) dans un récipient, la liaison réversible d'au moins un composant d'acide nucléique de l'échantillon biologique à au moins une particule paramagnétique, ladite particule paramagnétique étant une particule d'oxyde de fer dans un environnement acide ;
(ii) la séparation du composant d'acide nucléique lié à au moins une particule paramagnétique des composants non liés de l'échantillon biologique ;
(iii) le lavage du composant d'acide nucléique lié à au moins une particule paramagnétique ;
(iv) la séparation du composant d'acide nucléique lié à au moins une particule paramagnétique de la solution de lavage ;
(v) le retrait de l'au moins un composant d'acide nucléique de l'au moins une particule paramagnétique par un procédé d'élution et de neutralisation en deux étapes comprenant le fait
- d'éluer le composant d'acide nucléique lié à au moins une particule paramagnétique avec une solution tampon d'élution à pH basique constituée d'hydroxyde de potassium ou d'hydroxyde de sodium, ce qui permet d'obtenir un échantillon élué ; et
- de neutraliser par la suite l'échantillon élué par ajout ultérieur d'un tampon de neutralisation choisi parmi la bicine, le Tris, l'acide 2-(cyclohexyl-amino)éthanesulfonique (CHES), l'acide N,N-bis(2-hydroxyéthyl)-2-amino-éthanesulfonique (BES), l'acide 4-morpholinepropanesulfonique (MOPS) ou le phosphate, ce qui permet d'obtenir un tampon optimisé,
(vi) puis la séparation et le retrait des particules paramagnétiques après neutralisation de l'échantillon tandis que la solution à pH optimisé contenant l'acide nucléique non lié est transférée pour une manipulation ultérieure,
où l'étape de retrait est séparée de l'étape de neutralisation.

2. Procédé de la revendication 1, dans lequel l'échantillon biologique est un échantillon clinique, médico-légal ou environnemental.

3. Procédé de la revendication 2, dans lequel l'échantillon biologique est un échantillon environnemental comprenant du sol, de l'eau, de l'air, des effluents en suspension ou de la poudre.

4. Procédé de la revendication 1, dans lequel l'échantillon biologique est prétraité pour lyser des cellules.

5. Procédé de la revendication 1, dans lequel ladite élution comprend l'élévation du pH avec le tampon d'élution à pH.

6. Procédé de la revendication 1, dans lequel le tampon d'élution a un pH compris entre 8 et 14.

7. Procédé de la revendication 1, dans lequel ledit tampon de neutralisation diminue le pH.

8. Procédé de la revendication 7, dans lequel le pH est compris entre 6 et 9, de préférence dans lequel le pH est compris entre 8 et 8,5, plus préférablement dans lequel le pH est de 8,4 après l'ajout du tampon de neutralisation.
